# EUROPÄISCHE PATENTSCHRIFT

(11) **EP 1 089 739 B1**
(45) Veröffentlichungstag und Bekanntmachung des Hinweises auf die Patenterteilung: **23.11.2005**
(21) Anmeldenummer: 99927902.9
(22) Anmeldetag: 08.06.1999
(51) Int. Cl.: A61K 31/675, A61K 9/28

(54) **CYCLOPHOSPHAMID FILMTABLETTEN**
CYCLOPHOSPHAMIDE COATED TABLETS
COMPRIMES DE CYCLOPHOSPHAMIDE POURVUS D'UN FILM

(30) Priorität: 15.06.1998 DE 19826517
(43) Veröffentlichungstag der Anmeldung: 11.04.2001
(73) Patentinhaber: Baxter Healthcare SA, 8304 Wallisellen (CH); Baxter International Inc., Deerfield, IL 60015 (US)
(72) Erfinder: ENGEL, Jürgen, D-63755 Alzenau (DE); RAWERT, Jürgen, D-63755 Alzenau (DE); SAUERBIER, Dieter, D-33824 Werther (DE); WICHERT, Burkhard, D-33615 Bielefeld (DE)
(74) Vertreter: Müller-Boré & Partner Patentanwälte
(86) Internationale Anmeldenummer: PCT/EP1999/003920
(87) Internationale Veröffentlichungsnummer: WO 1999/065499

(56) Entgegenhaltungen:
- EP-A- 0 519 099

## Beschreibung

Die Erfindung betrifft Cyclophosphamid Filmtabletten sowie ein Verfahren zu ihrer Herstellung. Die Erfindung ist in der pharmazeutischen Industrie anwendbar.

Cyclophosphamid ist ein seit Jahrzehnten in der Chemotherapie eingeführtes Mittel mit breitem Antitumorspektrum zur Behandlung solider Tumoren wie Mamma-Ca, Bronchial-Ca sowie Hämoblastosen.

An Arzneiformen sind bisher Tabletten, Dragees sowie hauptsächlich Lyophilsate mit verschiedenen Hilfsstoffen wie Mannit oder Harnstoff bekannt.

Das EP 0519099 beschreibt Tabletten enthaltend Cyclophosphamid und vorgequollene Stärke, hergestellt durch ein Direkt-Tablettierverfahren.

Da Cyclophosphamid gesundheitsgefährend ist und aus diesem Grund ein direkter Kontakt mit diesem Stoff ein potentielles Risiko darstellt, werden die nach EP 0519099 hergestellten Tabletten als Kerne für Manteltabletten verwendet und so mittels einer zweiten Tablettierung umhüllt. Dieses Verfahren ist technisch aufwendig. Ferner werden für die Herstellung von Manteltabletten spezielle Tablettiermaschinen benötigt.

Es besteht somit der Bedarf einer einfachen und wirtschaftlichen Herstellung von Cyclophosphamid enthaltenden, festen Arzneiform zur oralen Anwendung.

Hierbei gilt es zu berücksichtigen, daß die Arzneiformen umhüllt sein müssen, damit der direkte Kontakt zum cytotoxischen Wirkstoff vermieden wird.

Es ist außerdem bekannt, daß Cyclophosphamid chemisch labil ist, somit muß auch die Stabilität der Arzneiformen berücksichtigt werden.

Überraschenderweise ist es gelungen, Filmtabletten enthaltend Cyclophosphamid herzustellen, ohne den Einsatz von vorgequollener Stärke. Auf der Basis der in Beispiel I aufgeführten Kompatibilitätsuntersuchungen wurden geeignete Hilfsstoffe ausgewählt. Überraschend war hierbei, daß die Stabilität von Cyclophosphamid in Anwesenheit von vorgequollener Stärke eher mittelmäßig ist.

Außerdem war es überraschend, daß die gefertigten Filmtabletten eine ausreichende Stabilität zeigen, obwohl der Wirkstoff herstellungsbedingt während des Filmungsprozesses durch Feuchtigkeit und Wärme belastet wird.

Gemäß einem Aspekt der Erfindung werden Filmtabletten mit Cyclophosphamid als Wirkstoff, enthaltend im Kern Cyclophosphamid, einen oder mehrere Füllstoffe ausgewählt aus der Gruppe bestehend aus Lactose Monohydrat, D-Mannit und CaHPO₄, ein oder mehrere Trockenbindemittel ausgewählt aus der Gruppe bestehend aus nicht vorgequollener Maisstärke und mikrofeiner Cellulose, hochdisperses Siliciumdioxid als Fließregulierungsmittel, und Schmiermittel ausgewählt aus der Gruppe bestehend aus Magnesiumstearat, Stearinsäure, Glycinpalmitostearat, Polyethylenglycol, Talkum und Glycerinmonobehenat, wobei der Kern die Hilfsstoffe sowohl einzeln als auch im beliebigen Gemisch enthalten kann, bereitgestellt.

Gemäß einer bevorzugten Ausführungsform der Erfindung werden Filmtabletten wie oben beschrieben, enthaltend im Kern, pro 1 Teil Cyclophosphamid, Lactose Monohydrat, mikrofeine Cellulose, nicht vorgequollene Maisstärke, Talkum, hochdisperses Siliciumdioxid und Magnesiumstearat in folgendem Verhältnis: Lactose Monohydrate 0,2-1,5, vorzugsweise 0,5-1, besonders bevorzugt 0,73; Mikrofeine Cellulose 0,2-1,5, vorzugsweise 0,5-1, besonders bevorzugt 0,74; Nicht vorgequollene Maisstärke 0,1-1,5, vorzugsweise 0,2-0,7, besonders bevorzugt 0,37; Talkum 0,01-1,5, vorzugsweise 0,05-0,08, besonders bevorzugt 0,07; hochdisperses Siliciumdioxid 0,01-0,1, vorzugsweise 0,01-0,5, besonders bevorzugt 0,04; Magnesiumstearat 0,01-0,1, Vorzugsweise 0,01-0,05, besonders bevorzugt 0,03, bereitgestellt.

Gemäß einer weiteren bevorzugten Ausführungsform der Erfindung werden Filmtabletten wie vorstehend beschrieben, dadurch charakterisiert, dass der Kern 50,0 mg Cyclophosphamid (53,5 mg Cyclophosphamid Monohydrate), 39,0 mg Lactose Monohydrat, 20,0 mg nicht vorgequollene Maisstärke, 40,0 mg mikrofeine Cellulose, 2,0 mg hochdisperses Siliciumdioxid, 4,0 mg Talkum, und 1,5 mg Magnesiumstearat enthält, bereitgrestellt.

Gemäß einem weiteren Aspekt der Erfindung wird ein Verfahren zur Herstellung von Tablettenkemen geeignet zum Aufbringen von einem Filmüberzug, gekennzeichnet durch die Schritte, dass Cyclophosphamid, Lactose Monohydrat, mikrofeine Cellulose, nicht vorgequollene Maisstärke, Talkum und hochdisperses Siliciumdioxid gesiebt und homogenisiert werden, dann Magnesiumstearat zugefügt und vermischt wird, und die so erhaltene Masse zu Tablettenkernen verpreßt wird, bereitgestellt.

Gemäß einer bevorzugten Ausführungsform wird ein Verfahren wie vorstehend beschrieben, dadurch charakterisiert, dass im Kern, pro 1 Teil Cyclophosphamid, Lactose Monohydrat, mikrofeine Cellulose, nicht vorgequollene Maisstärke, Talkum, hochdisperses Siliciumdioxid und Magnesiumstearat im folgenden Verhältnis vorliegen: Lactose Monohydrate 0,2-1,5, vorzugsweise 0,5-1, besonders bevorzugt 0,73; Mikrofeine Cellulose 0,2-1,5, vorzugsweise 0,5-1, besonders bevorzugt 0,74; Nicht vorgequollene Maisstärke 0,1-1,5, vorzugsweise 0,2-0,7, besonders bevorzugt 0,37;Talkum 0,01-1,5, vorzugsweise 0,05-0,08, besonders bevorzugt 0,07; Hochdisperses Siliciumdioxid 0,01-0,1, vorzugsweise 0,01-0,5, besonders bevorzugt 0,04; Magnesiumstearat 0,01-0,1, Vorzugsweise 0,01-0,05, besonders bevorzugt 0,03, bereitgestellt.

Gemäß einer weiteren bevorzugten Ausführungsform wird ein Verfahren wie vorstehend beschrieben, dadurch charakterisiert, dass der Kern 50,0 mg Cyclosphophamid (53,5 mg Cyclophosphamid Monohydrat), 39,0 mg Lactose Monohydrat, 20,0 mg nicht vorgequollene Maisstärke, 40,0 mg mikrofeine Cellulose, 2,0 mg hochdisperses Siliciumdioxid, 4,0 mg Talkum, und 1,5 mg Magnesiumstearat enthält, bereitgestellt.

Gemäß einer weiteren bevorzugten Ausführungsform wird ein Verfahren zur Herstellung einer Filmtablette, dadurch charakterisiert, dass die wie vorstehend beschrieben hergestellten Tablettenkeme mit einer Suspension, erhalten durch Auflösen von 11,83 g Polyethylenglykol und 2,37 g Polysorbate 80 in 75,21 g Wasser, dann Lösen von 1,9 g Carboxymethylcellulose-Natrium in 80,0 g Wasser, dann Zusammenführen der beiden Lösungen, Hinzufügen von 23,67 g Talkum, 23, 67 g Titaniumdioxid und 0, 24 g Simeticone, dann Homogenisieren, anschließend Hinzugeben von 17,73 g einer 30 %igen Acrylsäureethylester-Methacrylsäure-Methylester-Copolymerisat-Dispersion, besprüht werden, bereitgestellt.

Gemäß einem weiteren Aspekt der Erfindung werden Tablettenkeme, erhalten gemäß dem Verfahren wie oben beschrieben, bereitgestellt.

Gemäß einem weiteren Aspekt der Erfindung werden Filmtabletten, erhalten gemäß dem Verfahren wie oben beschrieben, bereitgestellt.

### Beispiel 1

### Untersuchungen zur Kompatibilität von Cyclophosphamid mit diversen Tablettierhilfsstoffen

Es wurden jeweils 53,5 mg Cyclophosphamid und 86,5 mg (Hilfsstoff 1-10) bzw. 3,0 mg (Hilfsstoff 11-18) vermischt und komprimiert. Die Lagerung der Komprinate erfolgte bei 31° C über 6 Monate. Die Zersetzung des Wirkstoffes erfolgte über Chloridbestimmung.
In der folgenden Tabelle sind die Ergebnisse zusammengefaßt.

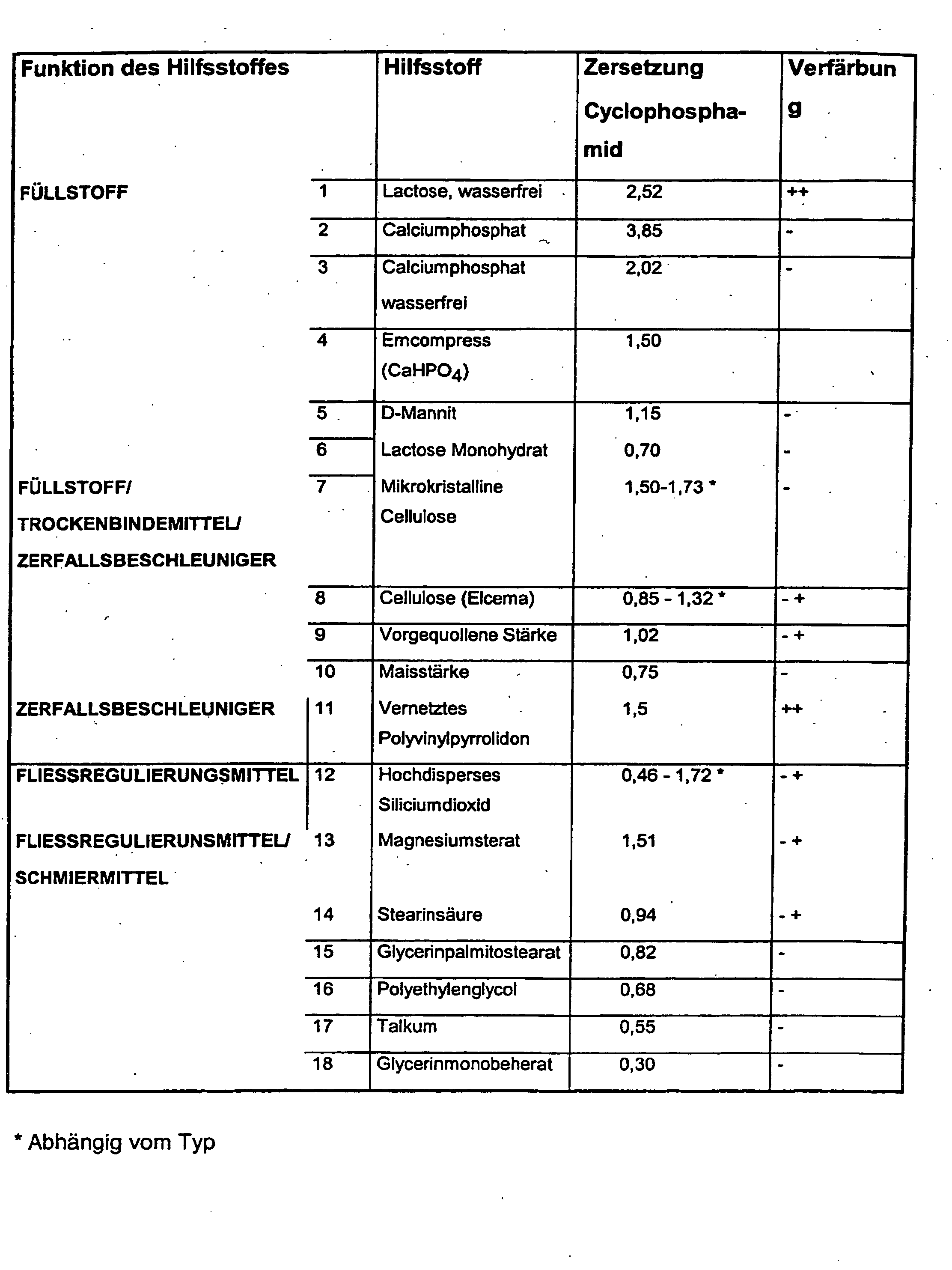

### Beispiel 2

### Herstellung von Tablettenkemen (50 mg Cyclophosphamid) Direkttablettierung

0,535 mg Cyclophosphamid, 0,390 mg Lactose Monohydrat, 0,400 mg microfeine Cellulose, 0,200 mg Maisstärke, 0,040 mg Talkum und 0,020 mg hochdisperses Siliciumdioxid werden gesiebt und homogenisiert. Anschließend wird 0, 015 mg Magnesiumstearat zugesetzt und gemischt. Die so hergestellte Masse wird zu Tabletten verarbeitet:
- Gewicht:: 160 mg
- Härte:: > 30 N
- Zerfall:: < 10 min.

### Beispiel 3

### Herstellung von Filmtabletten (50 mg Cyclophosphamid)

11,83 g Polyethylenglycol und 2,37 g Polysorbat 80 werden in 75,21 g Wasser gelöst.
1,9 g Carboxymethylcellulose Natrium werden in 80,0 g Wasser gelöst. Die Lösungen werden zusammengeführt. Es werden anschließend 23,67 g Talkum, 23,67 g Titandioxid und 0,24 g Simeticone hinzugefügt und homogenisiert. Anschließend werden 17,73 g einer 30%igen Acrylsäureethylester-Metharcrylsäure-Methylester-Copoylmerisat Dispersion in Wasser hinzugegeben.
Die Tablettenkeme werden alsdann in einem geeigneten Gerät mit der hergestellten Suspension besprüht:
- Sollgewicht einer Filmtablette:: 166 mg

### Beispiel 4

Untersuchung der Stabilität von Cyclophosphamid Filmtabletten

| **Zersetzung Cyclophosphamid nach 3 Monaten** | | |
|---|---|---|
| | 26° C / 60% rF | 31 ° C /40% |
| Charge 1 | 0,30 | 4,12 |
| Charge 2 | 0,17 | 2,36 |

Bei einer Lagerung bei < 25° C wird eine Stabilität der Filmtabletten von bis zu 3 Jahren erwartet.

## Patentansprüche

1. Filmtablette mit Cyclophosphamid als Wirkstoff, enthaltend im Kern Cyclophosphamid, einen oder mehrere Füllstoffe ausgewählt aus der Gruppe bestehend aus Lactose Monohydrat, D-Mannit und CaHPO₄, ein oder mehrere Trockenbindemittel ausgewählt aus der Gruppe bestehend aus nicht vorgequollener Maisstärke und mikrofeiner Cellulose, hochdisperses Siliciumdioxid als Fließregulierungsmittel, und Schmiermittel ausgewählt aus der Gruppe bestehend aus Magnesiumstearat, Stearinsäure, Glycinpalmitostearat, Polyethylenglycol, Talkum und Glycerinmonobehenat, wobei der Kern die Hilfsstoffe sowohl einzeln als auch im beliebigen Gemisch enthalten kann.

2. Filmtablette nach Anspruch 1, enthaltend im Kern, pro 1 Teil Cyclophosphamid, Lactose Monohydrat, mikrofeine Cellulose, nicht vorgequollene Maisstärke, Talkum, hochdisperses Siliciumdioxid und Magnesiumstearat in folgendem Verhältnis:
Lactose Monohydrate 0,2-1,5, vorzugsweise 0,5-1, besonders bevorzugt 0,73;
Mikrofeine Cellulose 0,2-1,5, vorzugsweise 0,5-1, besonders bevorzugt 0, 74;
Nicht vorgequollene Maisstärke 0,1-1,5, vorzugsweise 0,2-0,7, besonders bevorzugt 0,37;
Talkum 0,01-1,5, vorzugsweise 0,05-0,08, besonders bevorzugt 0,07;
Hochdisperses Siliciumdioxid 0,01-0,1, vorzugsweise 0,01-0,5, besonders bevorzugt 0,04;
Magnesiumstearat 0,01-0, 1, Vorzugsweise 0,01-0,05, besonders bevorzugt 0,03.

3. Filmtablette nach Anspruch 1 oder 2, **dadurch gekennzeichnet, dass** der Kern 50,0 mg Cyclosphophamid (53,5 mg Cyclophosphamid Monohydrat), 39,0 mg Lactose Monohydrat, 20,0 mg nicht vorgequollene Maisstärke, 40,0 mg mikrofeine Cellulose, 2,0 mg hochdisperses Siliciumdioxid, 4,0 mg Talkum, und 1,5 mg Magnesiumstearat enthält.

4. Verfahren zur Herstellung von Tablettenkernen geeignet zum Aufbringen von einem Filmüberzug, **gekennzeichnet durch** die Schritte, dass Cyclophosphamid, Lactose Monohydrat, mikrofeine Cellulose, nicht vorgequollene Maisstärke, Talkum und hochdisperses Siliciumdioxid gesiebt und homogenisiert werden, dann Magnesiumstearat zugefügt und vermischt wird, und die so erhaltene Masse zu Tablettenkemen verpreßt wird.

5. Verfahren nach Anspruch 4, **dadurch gekennzeichnet, dass** im Kern, pro 1 Teil Cyclophosphamid, Lactose Monohydrat, mikrofeine Cellulose, nicht vorgequollene Maisstärke, Talkum, hochdisperses Siliciumdioxid und Magnesiumstearat im folgenden Verhältnis vorliegen:
Lactose Monohydrat 0,2-1,5, vorzugsweise 0,5-1, besonders bevorzugt 0, 73;
Mikrofeine Cellulose 0,2-1,5, vorzugsweise 0,5-1, besonders bevorzugt 0,74;
Nicht vorgequollene Maisstärke 0,1-1,5, vorzugsweise 0,2-0,7, besonders bevorzugt 0,37;
Talkum 0,01-1,5, vorzugsweise 0,05-0,08, besonders bevorzugt 0,07;
Hochdisperses Siliciumdioxid 0,01-0,1, vorzugsweise 0,01-0,5, besonders bevorzugt 0,04;
Magnesiumstearat 0,01-0,1, Vorzugsweise 0,01-0,05, besonders bevorzugt 0,03.

6. Verfahren nach Anspruch 4 oder 5, **dadurch gekennzeichnet, dass** der Kern 50,0 mg Cyclophosphamid (53,5 mg Cyclophosphamid Monohydrat), 39,0 mg Lactose Monohydrat, 20,0 mg nicht vorgequollene Maisstärke, 40,0 mg mikrofeine Cellulose, 2,0 mg hochdisperses Siliciumdioxid, 4,0 mg Talkum, und 1,5 mg Magnesiumstearat enthält.

7. Verfahren zur Herstellung einer Filmtablette, **dadurch gekennzeichnet, dass** die nach einem der Ansprüche 4 bis 6 hergestellten Tablettenkerne mit einer Suspension, erhalten durch Auflösen von 11,83 g Polyethylenglykol und 2,37 g Polysorbate 80 in 75,21 g Wasser, dann Lösen von 1,9 g Carboxymethylcellulose-Natrium in 80,0 g Wasser, dann Zusammenführen der beiden Lösungen, Hinzufügen von 23,67 g Talkum, 23, 67 g Titaniumdioxid und 0, 24 g Simeticone, dann Homogenisieren, anschließend Hinzugeben von 17,73 g einer 30 %igen Acrylsäureethylester Methacrylsäure-Methylester-Copolymerisat-Dispersion, besprüht werden.

8. Tablettenkem, erhalten gemäß dem Verfahren nach einem der Ansprüche 4 bis 6.

9. Filmtablette, erhalten gemäß dem Verfahren nach Anspruch 7.

## Claims

1. A film-coated tablet with cyclophosphamide as active compound, comprising in the core cyclophosphamide, one or more fillers selected from the group consisting of lactose monohydrate, D-mannitol and CaHPO₄, one or more dry binders selected from the group consisting of nonpreswollen corn starch and microfine cellulose, highly disperse silica as flow regulator, and a lubricant selected from the group consisting of magnesium stearate, stearic acid, glycerol palmitostearate, polyethylene glycol, talcum and glycerol monobehenate, wherein the core can comprise the auxiliaries either individually or alternatively in any desired mixture.

2. The film-coated tablet according to claim 1, comprising, per 1 part of cyclophosphamide in the core, lactose monohydrate, microfine cellulose, nonpreswollen corn starch, talcum, highly disperse silica and magnesium stearate in the following ratio:
lactose monohydrate 0.2-1.5, preferably 0.5-1, particularly 0.73;
microfine cellulose 0.2-1.5, preferably 0.5-1, particularly 0.74;
nonpreswollen corn starch 0.1-1.5, preferably 0.2-0.7, particularly 0.37;
talcum 0.01-1.5, preferably 0.05-0.08, particularly 0.07;
highly disperse silica 0.01-0.1, preferably 0.01-0.5, particularly 0.04;
magnesium stearate 0.01-0.1, preferably 0.01-0.05, particularly 0.03.

3. The film-coated tablet according to claim 1 or 2, **characterized in that** the core comprises 50.0 mg cyclophosphamide (53.5 mg cyclophosphamide monohydrate), 39.0 mg lactose monohydrate, 20.0 mg nonpreswollen corn starch, 40.0 mg microfine cellulose, 2.0 mg highly disperse silica, 4.0 mg talcum, and 1.5 mg magnesium stearate.

4. A method for manufacturing of tablet cores suitable to be provided with a film coat, **characterized by** the steps that cyclophosphamide, lactose monohydrate, microfine cellulose, nonpreswollen corn starch, talcum and highly disperse silica are sieved and homogenized, then magnesium stearate is added and mixed, and the so obtained mass is pressed into tablet cores.

5. The method according to claim 4, **characterized in that** in the core the amount of lactose monohydrate, microfine cellulose, nonpreswollen corn starch, talcum, highly disperse silica and magnesium stearate, per 1 part of cyclophosphamide, is as follows:
lactose monohydrate 0.2-1.5, preferably 0.5-1, particularly 0.73;
microfine cellulose 0.2-1.5, preferably 0.5-1, particularly 0.74;
nonpreswollen corn starch 0.1-1.5, preferably 0.2-0.7, particularly 0.37;
talcum 0.01-1.5, preferably 0.05-0.08, particularly 0.07;
highly disperse silica 0.01-0.1, preferably 0.01-0.5, particularly 0.04;
magnesium stearate 0.01-0.1, preferably 0.01-0.05, particularly 0.03.

6. The method according to claim 4 or 5, wherein the core contains 50.0 mg cyclophosphamide (53.5 mg cyclophosphamide monohydrate), 39.0 mg lactose monohydrate, 20.0 mg nonpreswollen corn starch, 40.0 mg microfine cellulose, 2.0 mg highly dispersed silica, 4.0 mg talcum, and 1.5 mg magnesium stearate.

7. A process for manufacturing a film-coated tablet, **characterized in that** the tablet cores obtained according to anyone of claims 4 to 6 are sprayed with a suspension obtained by dissolving of 11.83 g polyethylene glycol and 2.37 g polysorbate 80 in water, further dissolving of 1.9 g carboxymethylcellulose sodium in 80.0 g water, then bringing the two solutions together, adding of 23.67 g talcum, 23.67 g titanium dioxide and 0.24 g simeticone thereto, then homogenizing, then adding of 17.73 g of a 30% ethyl acrylate-methyl methacrylate copolymer dispersion thereto.

8. A tablet core obtained by the process according to anyone of claims 4 to 6.

9. A film coated tablet obtained by the process according to claim 7.

## Revendications

1. Comprimé enrobé avec du cyclophosphamide comme principe actif, contenant dans le noyau du cyclophosphamide, une ou plusieurs charges choisies dans le groupe constitué du monohydrate de lactose, du D-mannitol et de CaHPO₄ ; un ou plusieurs liants secs choisis dans le groupe constitué de l'amidon de maïs non prétrempé et de la cellulose microfine ; du dioxyde de silicium hautement dispersé comme agent régulateur de flux et des agents lubrifiants choisis dans le groupe constitué du stéarate de magnésium, de l'acide stéarique, du palmitostéarate de glycine, du polyéthylèneglycol, du talc et du monobéhénate de glycérine, le noyau pouvant contenir les adjuvants seuls ou en mélanges quelconques.

2. Comprimé enrobé selon la revendication 1 contenant dans le noyau, pour 1 partie de cyclophosphamide, du monohydrate de lactose, de la cellulose microfine, de l'amidon de mais non prétrempé, du talc, du dioxyde de silicium hautement dispersé et du stéarate de magnésium dans les proportions suivantes :
monohydrate de lactose : 0,2 à 1,5, de préférence 0,5 à 1, tout particulièrement 0,73;
cellulose microfine : 0,2 à 1,5, de préférence 0,5 à 1, tout particulièrement 0,74;
amidon de mais non prétrempé : 0,1 à 1,5, de préférence 0,2 à 0,7, tout particulièrement 0,37;
talc : 0,01 à 1,5, de préférence 0,05 à 0,08, tout particulièrement 0,07;
dioxyde de silicum hautement dispersé : 0,01 à 0,1, de préférence 0,01 à 0,5, tout particulièrement 0,04; et
stéarate de magnésium : 0,01 à 0,1, de préférence 0,01 à 0,05, tout particulièrement 0,03.

3. Comprimé enrobé selon la revendication 1 ou 2, **caractérisé en ce que** le noyau contient 50,0 mg de cyclophosphamide (53,5 mg de monohydrate de cyclophosphamide), 39,0 mg de monohydrate de lactose, 20,0 mg d'amidon de maïs non prétrempé, 40,0 mg de cellulose microfine, 2,0 mg de dioxyde de silicium hautement dispersé, 4,0 mg de talc et 1,5 mg de stéarate de magnésium.

4. Procédé de fabrication de noyaux de comprimés, convenant à l'application d'un enrobage pelliculé, **caractérisé par** les étapes dans lesquelles du cyclophosphamide, du monohydrate de lactose, de la cellulose microfine, de l'amidon de mais non prétrempé, du talc et du dioxyde de silicium hautement dispersé sont tamisés et homogénéisés, puis du stéarate de magnésium est ajouté et mélangé et la masse ainsi obtenue est comprimée en noyaux de comprimés.

5. Procédé selon la revendication 4, **caractérisé en ce que** sont présents dans le noyau, pour 1 partie de cyclophosphamide, du monohydrate de lactose, de la cellulose microfine, de l'amidon de mais non prétrempé, du talc, du dioxyde de silicium hautement dispersé et du stéarate de magnésium dans les proportions suivantes :
monohydrate de lactose : 0,2 à 1,5, de préférence 0,5 à 1, tout particulièrement 0,73;
cellulose microfine : 0,2 à 1,5, de préférence 0,5 à 1, tout particulièrement 0,74;
amidon de maïs non prétrempé : 0,1 à 1,5, de préférence 0,2 à 0,7, tout particulièrement 0,37;
talc : 0,01 à 1,5, de préférence 0,05 à 0,08, tout particulièrement 0,07;
dioxyde de silicium hautement dispersé : 0,01 à 0,1, de préférence 0,01 à 0,5, tout particulièrement 0,04;
stéarate de magnésium : 0,01 à 0,1, de préférence 0,01 à 0,05, tout particulièrement 0,03.

6. Procédé selon la revendication 4 ou 5, **caractérisé en ce que** le noyau contient 50,0 mg de cyclophosphamide (53,5 mg de monohydrate de cyclophosphamide), 39,0 mg de monohydrate de lactose, 20,0 mg d'amidon de maïs non prétrempé, 40,0 mg de cellulose microfine, 2,0 mg de dioxyde de silicium hautement dispersé, 4,0 mg de talc et 1,5 mg de stéarate de magnésium.

7. Procédé de fabrication d'un comprimé enrobé, **caractérisé en ce que** les noyaux de comprimés fabriqués selon l'une quelconque des revendications 4 à 6 sont pulvérisés avec une suspension, obtenue par dissolution de 11,83 g de polyéthylèneglycol et 2,37 g de polysorbate 80 dans 75,21 g d'eau, suivie de la dissolution de 1,9 g de carboxyméthylcellulose sodique dans 80,0 g d'eau, suivie ensuite de la jonction des deux solutions, de l'addition de 23,67 g de talc, de 23,67 g de dioxyde de titane et de 0,24 g de siméticone, puis de l'homogénéisation et enfin de l'addition de 17,73 g d'une dispersion à 30% du copolymère d'acrylate d'éthyle et de méthacrylate de méthyle

8. Noyau de comprimé obtenu selon le procédé de l'une quelconque des revendications 4 à 6.

9. Comprimé enrobé obtenu selon le procédé de la revendication 7.
